# EUROPEAN PATENT APPLICATION

(11) **EP 4 613 227 A1**
(43) Date of publication of application: **10.09.2025**
(21) Application number: 23886107.4
(22) Date of filing: 23.10.2023
(51) Int. Cl.: A61B 34/30, A61B 34/00, A61B 17/16, A61B 90/00, A61B 17/00

(54) **SURGICAL TOOL RETAINING DEVICE FOR SURGICAL ROBOT**

(30) Priority: 04.11.2022 KR 20220146261
(71) Applicant: Curexo Inc., Seoul 05814 (KR)
(72) Inventor: LEE, Chang Hun, Seoul 05814 (KR); LEE, Kwan Ju, Seoul 05814 (KR)
(74) Representative: Stöckeler, Ferdinand
(86) International application number: PCT/KR2023/016444
(87) International publication number: WO 2024/096411

(57) **Abstract**

The disclosure relates to a surgical tool-fixing device for a surgical robot, and more particularly to a surgical tool-fixing device for a surgical robot, which stably holds and supports a sleeve for supporting a shaft of a surgical tool, thereby preventing deformation of related parts and improving durability.

The surgical tool-fixing device for a surgical robot includes: a sleeve supporting a surgical tool including a shaft; a chuck device part including a chuck body to which the sleeve is coupled, chucks selectively provided inside the chuck body and selectively clamping the shaft to hold the surgical tool, and an operation force applying portion applying operation forces for clamping and unclamping operations of the chucks; and a holder part installing the chuck device part thereon, mounted to a distal end of a robot arm, and including a sleeve supporting portion holding and supporting the sleeve not to shake, wherein the sleeve includes a first sleeve fastening portion formed in a direction facing the sleeve supporting portion of a hollow sleeve body formed with a shaft insertion hole, and the sleeve supporting portion is formed with a second sleeve fastening portion to couple with the first sleeve fastening portion.

## Description

### [TECHNICAL FIELD]

The disclosure relates to a surgical tool fixing device for a surgical robot, and more particularly to a surgical tool-fixing device for a surgical robot, which stably holds and supports a sleeve for supporting a shaft of a surgical tool, thereby preventing deformation of related parts and improving durability.

### [BACKGROUND ART]

In general, surgical treatment of joint diseases includes arthroscopic surgery, cartilage cell transplantation, etc., and artificial joint surgery is performed in the case of severe diseases. Artificial joint surgery representatively includes manual artificial joint surgery performed by medical personnel, and artificial joint surgery using a robot.

Artificial joint surgery using the robot refers to a surgical procedure that cuts off the kneecap and installs an artificial knee joint (implant) by rotating a cutter of a cutting device mounted to a distal end of a position-variable arm of the robot based on information input to a computer, and employs a cutting device including a cutting tool such as a drill (hereinafter referred to as a 'drill').

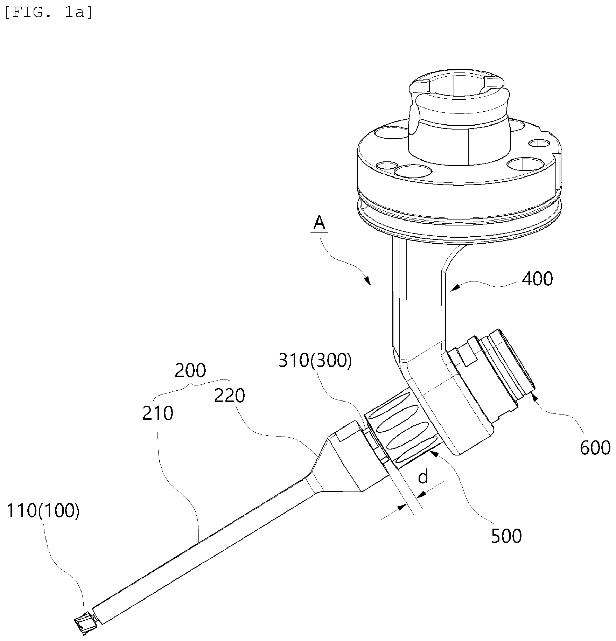

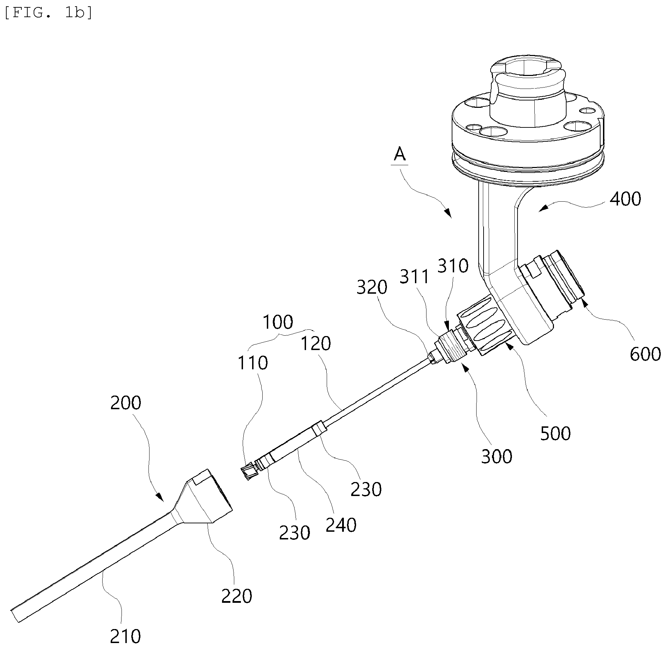

FIG. 1A is a perspective view showing an assembled state to illustrate a conventional surgical tool-fixing device for an orthopedic surgical robot, and FIG. 1B is an exploded perspective view to illustrate the conventional surgical tool-fixing device for the orthopedic surgical robot.

Referring to FIGS. 1A and 1B, a conventional surgical tool-fixing device A for an orthopedic surgical robot includes a sleeve 200 supporting a drill 100 that performs a cutting operation, a chuck device part 300 holding the drill, a holder part 400 installed with the chuck device part and mounted to the distal end of the robot's position-variable arm (not shown), an operating nut 500 applying an operation force to make the drill be clamped to or unclamped from the chuck device part 300, and a motor (not shown) mounted to a motor coupling member 600 connected to the rear of the holder portion 400 to provide a rotational force to the drill.

In more detail, the drill 100 includes a head 110 formed with cutting edges, and a shaft 120 formed extending from the head 110 and shaped like a round bar. The drill 100 performs the cutting operation as the rear end of the shaft 120 is connected to a motor and rotated, the outer circumferential surface of the shaft 120 is rotatably supported by the sleeve 200 so that the shaft 120 is prevented from vibration or bending when rotating, and the head 110 protrudes outward from the sleeve 200 so that the cutting operation for a bone can be performed by the rotation of the head 110.

The sleeve 200 includes a pipe portion 210 having a small diameter, and a fastening cap 220 formed on one end of the pipe portion so as to be fastened to the chuck device part 300. In this sleeve, a bearing 230 rotatably supporting the shaft of the drill, a spacer 240, etc., are inserted.

Further, the fastening cap 220 of the sleeve 200 is formed with a female screw thread (not shown) on the inner surface of the hollow thereof, and the female screw thread of the fastening cap 220 is fastened to a male thread portion 311 formed on a chuck body 310.

The chuck device part 300 refers to a component to perform a clamping operation or an unclamping operation by pressing the shaft of the cutter as chucks 320 (also referred to as collets, a part to hold the shaft of the drill, usually 3 to 4 jaws that moves radially) provided in the front of the chuck body 310 are brought together or separated, and may employ a medical chuck device used in orthopedics, dentistry, etc. among the well-known chuck devices. In addition, the chuck body 310 is formed with an operating force applying portion (not shown) such as an adjusting screw portion to which the operating nut 500 is fastened, thereby pressurizing and holding the shaft as the chucks are brought together when the operating nut 500 rotates in a certain direction, and releasing the holding state of the shaft as the chucks are spread when the operating nut 500 rotates in the reverse direction.

The foregoing conventional surgical tool-fixing device for the orthopedic surgical robot performs a cutting operation while supporting the drill 100, but has limitations that result in the following problems.

In the conventional surgical tool-fixing device A for an orthopedic surgical robot, a large load acting on the left and right sides is transmitted to the sleeve 200 while the drill 100 is moved leftwards and rightwards during the cutting process, and the sleeve 200 is slightly deformed when receiving the large load on the left and right sides because the sleeve 200 has a long cantilever structure screw-coupled to the chuck body 310 at a separation distance d without a separate support means. The deformation of the sleeve 200 causes the shaft 120 of the drill 100 and related parts (or bearings) to be deformed, thereby reducing the durability. Further, the chuck device part 300 to which the shaft 120 is clamped is also deformed, thereby reducing durability. In particular, the female thread portion of the sleeve 200 is screw-coupled to the front male thread portion 311 of the chuck body 310, and thus there is a problem in that external force repeatedly transmitted from the sleeve 200 causes the chuck device part 300 to be not only damaged or decreased in lifespan but also malfunctioning. Thus, there are disadvantages such as increased maintenance costs due to frequent replacement of parts, impossibility of stable surgery, increased concern about negligent accidents, and increased downtime of surgery.

In addition, when the conventional surgical tool-fixing device for an orthopedic surgical robot is to be disassembled for cleaning or replacing the drill 100, the operating nut 500 should be rotated to release the holding state of the shaft 120 from the chuck device part 300 or to apply a tightening force. However, it is very difficult for a user who has a weak grip, such as a female nurse, to manipulate the operating nut 500 or make sure the holding state, thereby causing a mal-operation or a negligent accident.

Further, the conventional surgical tool-fixing device for an orthopedic surgical robot has problems making preparation and use difficult and increasing medical costs due to increased manufacturing costs because the sleeve suitable for a cutter (or drill) should be individually manufactured when the length or diameter of that cutter is changed according to the methods or types of artificial joint surgery.

### [DOCUMENT OF RELATED ART]

### [PATENT DOCUMENT]

(Patent Document 1) Korean Patent No. 10-2359592, titled "HOLDER FOR EASY JOIN OF END EFFECTOR"
(Patent Document 2) Japanese Patent No. 6385275, titled "SURGICAL INSTRUMENT COMPRISING A CUTTING ACCESSORY EXTENDING FROM THE HOUSING AND AN ACTUATOR FOR ESTABLISHING THE POSITION OF THE CUTTING ACCESSORY RELATIVE TO THE HOUSING" [DISCLOSURE]

### [TECHNICAL PROBLEM]

The disclosure has been conceived from the foregoing, and an aspect of the disclosure is to provide a surgical tool-fixing device for a surgical robot, which stably holds and supports a sleeve for supporting a shaft of a surgical tool, thereby preventing deformation of related parts and improving durability.

In more detail, an aspect of the disclosure is to provide a surgical tool-fixing device for a surgical robot, in which a sleeve is coupled to not a chuck device part but a sleeve supporting portion extending from a holder part, thereby preventing damage to or deformation of the chuck device part, improving durability, and improving operational stability of the chuck device part.

Further, the disclosure is to provide a surgical tool-fixing device for a surgical robot, in which separating and mounting operations for a surgical tool are conveniently and stably performed with little effort.

Besides, the disclosure is to provide a surgical tool-fixing device for a surgical robot, which allows various medical tools to be mounted and used interchangeably, and is convenient to install a calibration tool to perform accurate calibration.

### [TECHNICAL SOLUTION]

According to an embodiment of the disclosure, a surgical tool-fixing device for a surgical robot, includes: a sleeve supporting a surgical tool including a shaft; a chuck device part including a chuck body to which the sleeve is coupled, chucks selectively provided inside the chuck body and selectively clamping the shaft to hold the surgical tool, and an operation force applying portion applying operation forces for clamping and unclamping operations of the chucks; and a holder part installing the chuck device part thereon, mounted to a distal end of a robot arm, and including a sleeve supporting portion holding and supporting the sleeve not to shake, wherein the sleeve includes a first sleeve fastening portion formed in a direction facing the sleeve supporting portion of a hollow sleeve body formed with a shaft insertion hole and the sleeve supporting portion is formed with a second sleeve fastening portion to couple with the first sleeve fastening portion.

The first sleeve fastening portion may include a sleeve male thread portion including a plurality of male threads formed on an outer circumferential surface of a thread-formed hollow body integrally formed on a first side of the hollow sleeve body.

The sleeve supporting portion may include a supporting protrusion extending from the holder part, and a sleeve mounting portion formed at the end portion of the supporting protrusion.

The second sleeve fastening portion may include a holder female thread portion formed with a plurality of female threads on the sleeve mounting portion to couple with the sleeve male thread portion.

The supporting protrusion may protrude forward to provide an operational space inside which the operation force applying portion is positioned, and the sleeve mounting portion may be formed with the holder female thread portion on an inner circumferential surface of a hollow hole of a conical body with a small front outer diameter and a large rear outer diameter.

The hollow sleeve body may be structured to include a first hollow sleeve body formed with the first sleeve fastening portion at an end portion thereof, and a second hollow sleeve body extending in a rod-shaped structure with a smaller outer diameter than the first hollow sleeve body, and the surgical tool may include a reamer with a reamer basket-cup formed at an end portion of the shaft to be inserted in the shaft insertion hole and performing a cutting operation.

The surgical tool-fixing device may further include a calibration mounting part that includes a holding cap member fastened to the hollow sleeve body to install a calibration tool, and a calibration mounting member mounted to the sleeve by the holding cap member.

The calibration mounting member may include a bar-shaped calibration shaft in which a marker is installed, and a position-setting member formed in the calibration shaft and seated on the holding cap member.

The holding cap member may include a cap body shaped like a cap, a rod insertion hole formed in the cap body to insert the calibration shaft therein, and a cap female thread formed on an inner circumferential surface of the cap body to be fastened to a cap-coupling male thread formed on a second side of the hollow sleeve body, and the surgical tool-fixing device may further include a mounting member rotation blocking portion configured to block movement of the calibration mounting member.

The surgical tool may include a plurality of cutters including a cutter head with a plurality of blades on an outer end portion of the shaft, in which the cutter having a head diameter of 3.2 mm and the cutter having a head diameter of 5.0 mm are formed to have a protrusion length of 40 mm to 70 mm from a free end of the sleeve in a mounted state, and the cutter having a head diameter of 6.2 mm is formed to have a protrusion length of 70 mm to 85 mm.

The surgical tool-fixing device may further include an operating handle connecting with the operation force applying portion and applying an operating force, wherein the operating handle includes an engagement portion placed in the operational space to engage with the operation force applying portion, and a handle unit extending from the engagement portion.

The handle unit may include a first handle unit including a first engagement portion to engage with an outer surface of the operation force applying portion on a first side, and first handles extending from both ends of the first engagement portion and formed with fastening holes; a second handle unit including a second engagement portion to engage with an outer surface of the operation force applying portion on a second side, and second handles extending from both ends of the second engagement portion and formed with fastening holes; and fastening members to be fastened to the fastening holes of the first and second handle units, so as to be placed and assembled inside the operational space.

### [ADVANTAGEOUS EFFECTS]

With a surgical tool-fixing device for a surgical robot according to the disclosure, the sleeve is not conventionally screw-coupled to the chuck device part, but the first sleeve fastening portion formed in the sleeve is directly screw-coupled to the holder part, thereby not only maintaining a firmly holding state but also fundamentally preventing an external force from being applied to the chuck device part. Accordingly, the shaft of the cutter, the built-in bearings of the sleeve, etc. are prevented from deformation, and the chuck device part to which the shaft is clamped is also prevented from deformation and improved in durability, thereby having effects on reducing maintenance costs, performing surgery stably, lowering a risk of negligent accidents, and shortening downtime of surgery.

In addition, with a surgical tool-fixing device for a surgical robot according to the disclosure, when the surgical tool is to be disassembled and assembled, a user can easily perform the clamping operation and the unclamping operation for the surgical tool by rotating the operating handle forwards and reversely, and even a user who has a weak grip can conveniently perform the operations without difficulty.

Further, a surgical tool-fixing device for a surgical robot according to the disclosure has an effect on easily replacing and using various cutters because a sleeve includes a hollow sleeve body having a relatively large outer diameter rather than a conventional thin and long pipe and is thus interchangeably used without being replaced every time when the type of cutter is changed.

Besides, a surgical tool-fixing device for a surgical robot according to the disclosure has an effect on performing simple and accurate calibration by assembling a calibration mounting part to a sleeve through a fastening method.

### [DESCRIPTION OF DRAWINGS]

FIG. 1A is a perspective view showing an assembled state to illustrate a conventional surgical tool-fixing device for an orthopedic surgical robot.
FIG. 1B is an exploded perspective view to illustrate the conventional surgical tool-fixing device for the orthopedic surgical robot.
FIG. 2 is a perspective view of a surgical tool-fixing device for a surgical robot according to a first embodiment of the disclosure.
FIG. 3 is an exploded perspective view of the surgical tool-fixing device for a surgical robot according to the first embodiment of the disclosure.
FIGS. 4A to 4D are views to illustrate the main components of the surgical tool-fixing device for a surgical robot according to the first embodiment of the disclosure, in which FIG. 4A is a partially exploded perspective view of a chuck device part and a holder part, FIG. 4B is a partially exploded perspective view of a sleeve and a sleeve supporting portion in the holder part, FIG. 4C is a partially exploded perspective view of an operating handle, and FIG. 4D is a perspective view of a motor connecting member.
FIG. 5 is a view to illustrate the operating state of the surgical tool-fixing device for a surgical robot according to the first embodiment of the disclosure.
FIGS. 6A to 6C are views showing cutters applied as a surgical tool to the surgical tool-fixing device for a surgical robot according to the first embodiment of the disclosure, in which FIG. 6A shows the cutter having a head diameter of 3.2 mm, FIG. 6B shows the cutter having a head diameter of 5.0 mm, and FIG. 6C shows the cutter having a head diameter of 6.2 mm.
FIGS. 7A and 7B are perspective views showing states that a cutter applied as a surgical tool to the surgical tool-fixing device for a surgical robot according to the first embodiment of the disclosure, in which FIG. 7A shows an installation state of the cutter having a head diameter of 5.0 mm, and FIG. 7B shows an installation state of the cutter having a head diameter of 6.2 mm.
FIGS. 8A to 8C are views showing states that a calibration tool is installed as a surgical tool in the surgical tool-fixing device for a surgical robot according to the first embodiment of the disclosure, in which FIG. 8A is a perspective view, FIG. 8B is a schematic exploded perspective view, and FIG. 8C is an exploded perspective view of a main component.
FIGS. 9A to 9C are views to illustrate a calibration mounting part installed in the surgical tool-fixing device for a surgical robot according to the first embodiment of the disclosure, in which FIG. 9A is a partial perspective view, FIG. 9B is a partially exploded perspective view, and FIG. 9C is a partial cross-sectional view.
FIGS. 10A and 10B are views to illustrate a surgical tool-fixing device for a surgical robot according to a second embodiment of the disclosure, in which FIG. 10A is a perspective view, and FIG. 10B is an exploded perspective view.
FIGS. 11A and 11B are views to illustrate a surgical tool-fixing device for a surgical robot according to a third embodiment of the disclosure, in which FIG. 11A is a perspective view, and FIG. 11B is an exploded perspective view of a sleeve portion having distinctive features.
FIG. 11C is a view showing a state in which a calibration tool is mounted to a sleeve of the surgical tool-fixing device for a surgical robot according to the second embodiment of the disclosure.

### [BEST MODE]

Below, exemplary embodiments of the disclosure will be described in detail with reference to the accompanying drawings, in which the same numerals refer to the same components throughout.

Meanwhile, detailed descriptions of the configurations and their operations and effects, which can be easily understood by a person having ordinary knowledge in the art from general technologies in the accompanying drawings, will be simplified or omitted. Further, the disclosure is characterized in a surgical tool-fixing device for a surgical robot, and thus related parts will be illustrated and described, and the descriptions about the remaining parts will be simplified or omitted.

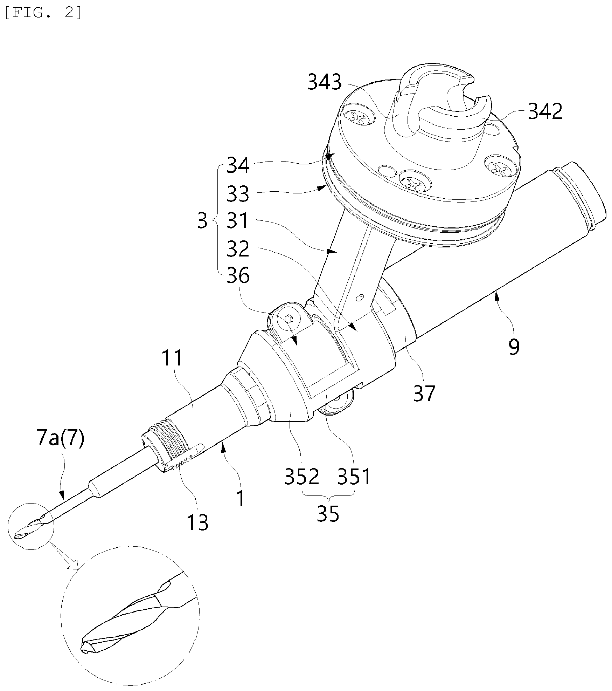

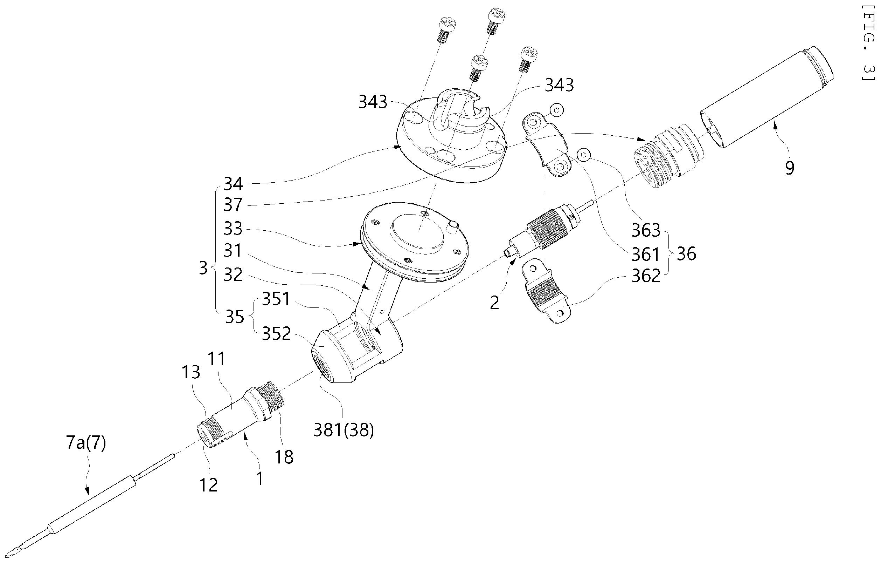

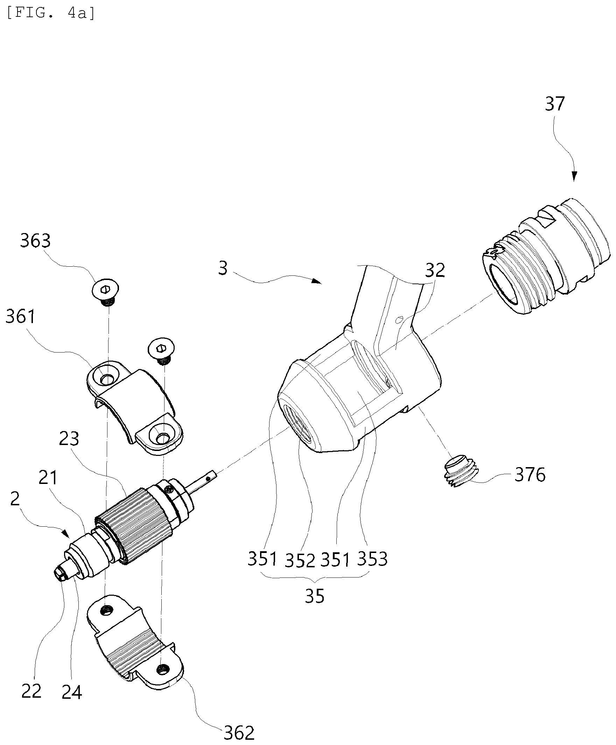

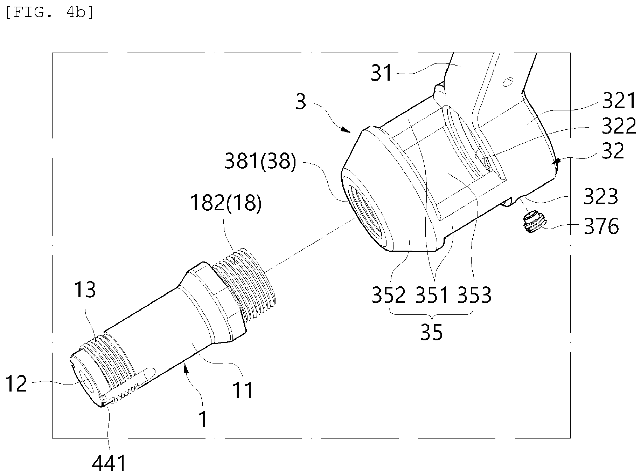

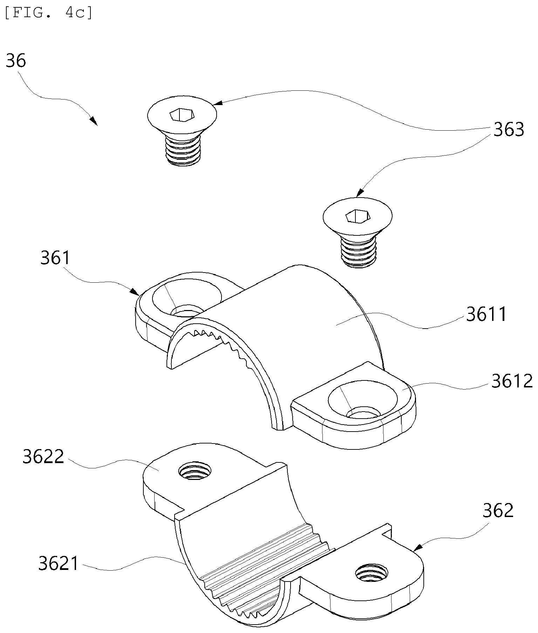

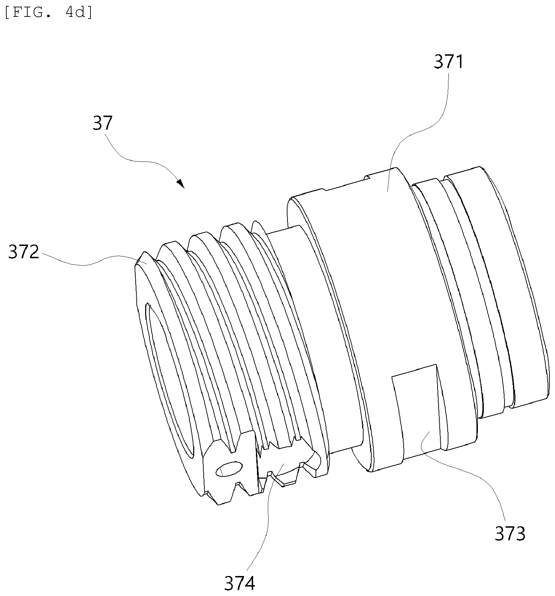

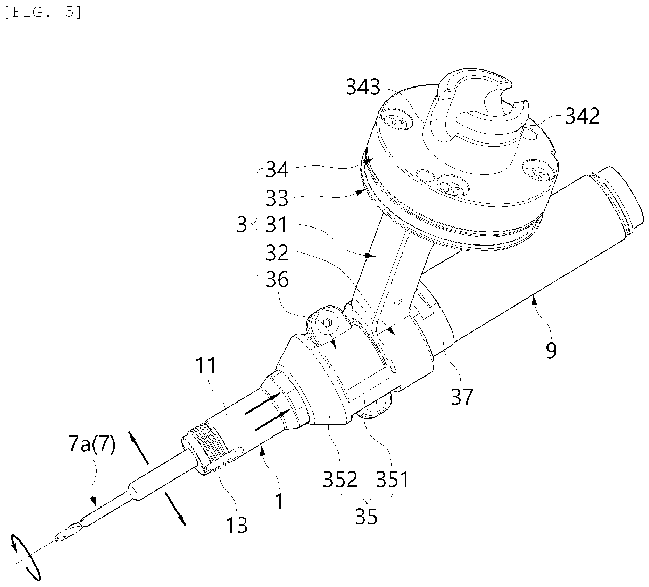

FIG. 2 is a perspective view of a surgical tool-fixing device for a surgical robot according to a first embodiment of the disclosure, FIG. 3 is an exploded perspective view of the surgical tool-fixing device for a surgical robot according to the first embodiment of the disclosure, and FIGS. 4A to 4D are views to illustrate the main components of the surgical tool-fixing device for a surgical robot according to the first embodiment of the disclosure, in which FIG. 4A is a partially exploded perspective view of a chuck device part and a holder part, FIG. 4B is a partially exploded perspective view of a sleeve and a sleeve supporting portion in the holder part, FIG. 4C is a partially exploded perspective view of an operating handle, and FIG. 4D is a perspective view of a motor connecting member. FIG. 5 is a view to illustrate an operating state of the surgical tool-fixing device for a surgical robot according to the first embodiment of the disclosure.

Referring to FIGS. 2 to 5, the surgical tool-fixing device for a surgical robot according to the first embodiment of the disclosure includes a sleeve 1, a chuck device part 2, and a holder part 3 to hold and use a surgical tool, and is characterized in that the sleeve 1 is not conventionally coupled to the chuck device part 2 but directly coupled to a supporting body, i.e., the holder part 3.

To this end, the sleeve 1 includes a first sleeve fastening portion 18 for coupling with the holder part 3, and the holder part 3 includes a sleeve supporting portion 35 having a second sleeve fastening portion 38 to which the first sleeve fastening portion 18 is coupled.

In more detail, the first sleeve fastening portion 18 is formed in a direction facing the sleeve supporting portion 35 of a hollow sleeve body 11 (to be described later), and is formed with a thread-formed hollow body integrally formed on one side of the hollow sleeve body, and a sleeve male thread portion 182 having a plurality of male threads formed on the outer circumferential surface of the thread-formed hollow body.

The second sleeve fastening portion 38 includes a holder female thread portion 381 formed with a plurality of female threads on the inner circumferential surface of a central hole of a sleeve mounting portion 352 so as to couple with the sleeve male thread portion 182.

The sleeve supporting portion 35 formed with the second sleeve fastening portion 38 is formed at an end portion of a supporting protrusion 351 extending from the holder part 3, which will be described in detail below.

The sleeve 1 refers to a component that supports various surgical tools 7 having a shaft 71, 421, such as a cutter 7a or a calibration tool 7b (to be described later), and is characterized in supporting various medical tools interchangeably unlike a conventional long and thin pipe that lacks compatibility and has weak bending rigidity.

The sleeve 1 includes a hollow sleeve body 11 shaped like a roughly round bar, which has a shaft insertion hole 12 formed penetrating the hollow sleeve body 11 along a longitudinal direction, the foregoing first sleeve fastening portion 18 formed in the hollow sleeve body 11 on one side, and a cap fastening screw portion 13 formed on the outer circumferential surface of the other side.

In addition, the hollow sleeve body 11 is provided with a plurality of support bearings (not shown) installed inside the shaft insertion hole 12 to support the shaft, and a spacer (not shown) installed being in contact with the support bearings and formed having a cylindrical shape. For example, the support bearings (not shown) may include ball bearings inserted forward and backward with the spacer therebetween to support the outer and inner sides of the shaft 71.

The chuck device part 2 refers to a component to which the sleeve 1 including the shaft is fastened, and performs a clamping operation or an unclamping operation by pressing the shaft 71 of the surgical tool as the chuck 22 provided in the front of the chuck body 21 is brought together or spread.

In addition, the chuck device part 2 includes the chuck body 21 to which the sleeve 1 is coupled, the chuck 22 provided inside the chuck body 21 and selectively clamping the shaft 71 of the cutter 7a to clamp the surgical tool, and an operation force applying portion 23 applying operation forces for the clamping and unclamping operations of the chuck 22, and has a structural distinction that the male thread portion 311 (see FIG. 1B) conventionally formed in the chuck body 21 so as to couple with the sleeve is omitted.

Further, the chuck body 21 is internally provided with a chuck moving member 24 from which the chuck 22 appears and disappears by the force of an internal spring (not shown) as the operation force applying portion 23 rotates forward and backward inside a round bar-shaped body approximately having a hollow. In more detail, when the operation force applying portion 23 rotates forward, the chuck 22 moves rearward and is brought together to press and clamp the shaft. When the operation force applying portion 23 rotates backward, the chucks move frontward and separate to unclamp the shaft.

In addition, the chuck device part 2 is applicable to medical chuck devices applicable to orthopedics, dentistry, etc. among the well-known chuck devices to couple with the sleeve, except the male thread portion 311 (see FIG. 1B) conventionally formed in the chuck body 21 to couple with the sleeve, and thus detailed descriptions about specific configurations thereof will be omitted.

The holder part 3 refers to a component mounted to a distal end of a robot arm (not shown) and used to install the chuck device part 2 thereon, and includes a holder rod 31 roughly shaped like a rod, a holder head 32 formed below at a first end of the holder rod 31, a connection plate 33 formed above at a second end of the holder rod 31, an arm connection member 34 connected to the connection plate 33 for the connection with the distal end of the robot arm, a sleeve supporting portion 35 protruding forward to implement a function of supporting the sleeve 1, and an operating handle 36 provided inside the sleeve supporting portion 35.

The holder head 32 is formed as a ring body 321 having a connecting hole 322 into which a motor connecting member 37 (to be described later) is inserted, and includes a screw hole 323 perforated in the ring body 321 to communicate with the connecting hole 322, and the foregoing sleeve supporting portion 35 configured to protrude forward.

For example, the arm connection member 34 is configured as a clamp member to connect with a robot-side clamp member (not shown) mounted on the opposite robot arm (not shown), and the clamp member has a disc-shaped clamp body 341 formed with a plurality of fastening holes, and a clamp protrusion 342 formed with a locking hole 343 inserted in or coupled to a clamping shaft of the robot-side clamp member.

The sleeve supporting portion 35 refers to a component formed extending from the holder part 3 and holding and supporting the sleeve 1 not to shake, and includes the supporting protrusion 351 extending from the holder part 3, and a sleeve mounting portion 352 to which the sleeve male thread portion 182 of the first sleeve fastening portion 18 of the sleeve 1 is fastened.

The supporting protrusion 351 is formed protruding forward from the holder head 32 so as to provide an operational space 353 inside which the operation force applying portion 23 is positioned. Not to hinder a forward rotation of the operating handle 36 for the clamping operation of the chuck device part 3 and a reverse rotation of the operating handle 36 for the unclamping operation of the chuck device part 2, two pieces of supporting protrusions 351 may be formed at an angle of approximately 180° (i.e., an angle excluding the thickness of the supporting protrusion from 180°).

The sleeve mounting portion 352 is formed as a conical body with a small front outer diameter and a large rear outer diameter, and is formed with the holder female thread portion 381 configured as the second sleeve fastening portion 38 on the inner circumferential surface of the hollow hole formed therein.

The operating handle 36 refers to a component that is placed in the operational space 353 so as to connect with the operation force applying portion 23 and apply an operating force, and includes an engagement portion to engage with the operation force applying portion 23 and a handle unit protruding from the engagement portion.

In more detail, it is important that the operating handle 36 is configured to be disposed and assembled in the operational space 353 provided inside the sleeve supporting portion 35.

To this end, the operating handle 36 has a structure separable into a first handle unit 361, a second handle unit 362, and a fastening member 363 for fastening the first handle unit 361 and the second handle unit 362.

The first handle unit 361 includes a first engagement portion 3611 having a roughly hemispherical-shaped body and formed with a serration gear on the inner surface thereof to mesh with the outer surface of the operation force applying portion 23 on one side, and first handles 3612 extending from both ends of the first engagement portion 3611 and formed with fastening holes.

The second handle unit 362 includes a second engagement portion 3621 having a roughly hemispherical-shaped body and formed with a serration gear on the inner surface thereof to mesh with the outer surface of the operation force applying portion 23 on the other side, and second handle 3622 extending from both ends of the second engagement portion 3621 and formed with fastening holes.

The fastening member 363 refers to a member to be fastened to the fastening holes of the first handle unit 361 and the second handle unit 362, and is typically achieved by a screw.

Meanwhile, the chuck device part 2 described above includes the motor connecting member 37 coupled to the rear thereof and connecting with a motor 9.

The motor connecting member 37 includes a thread portion 372 and a fastening groove 373 formed on the outer surface of a rounded bar-shaped body 371 having a hollow hole inside which the shaft of the cutter is placed, and a thread groove 374 recessed on the thread portion 372 to accommodate an end portion of a screw 376 inserted through the screw hole 323 of the holder head 32.

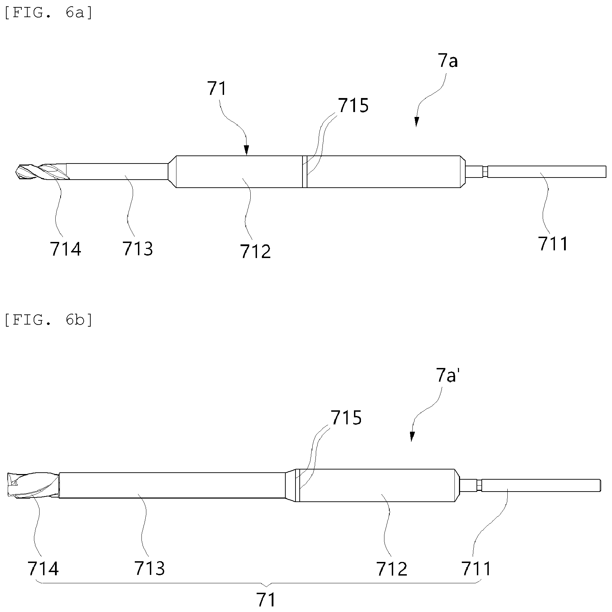

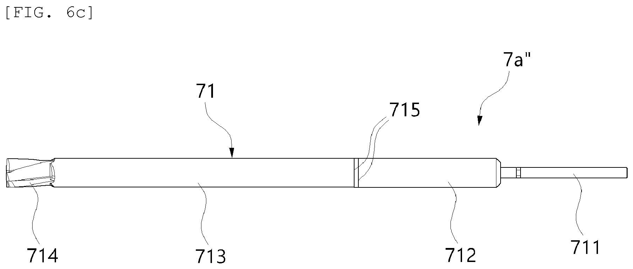

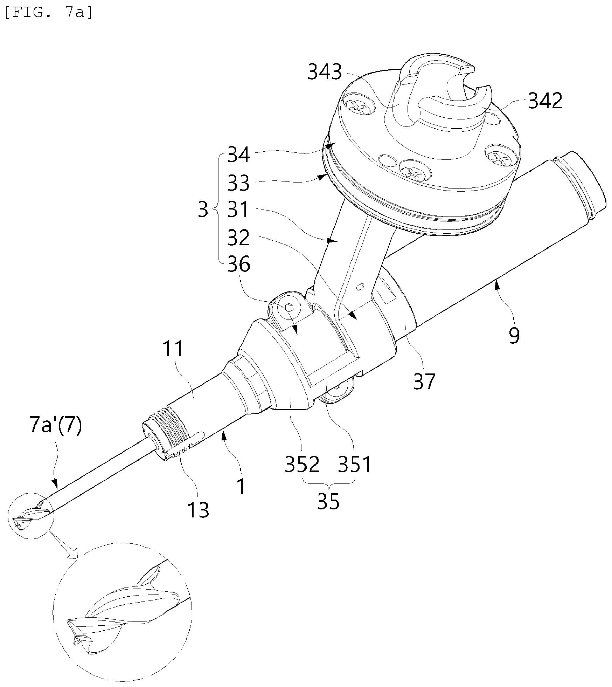

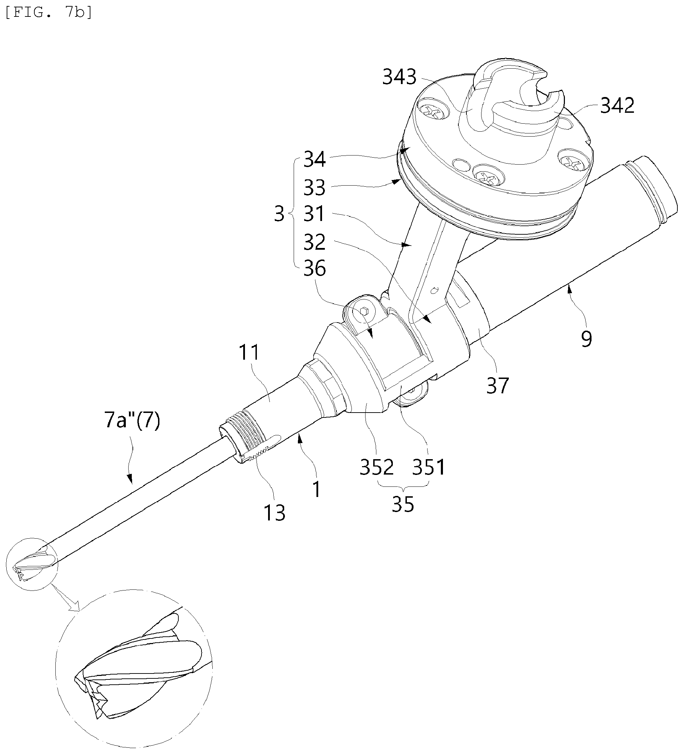

FIGS. 6A to 6C are views showing cutters applied as a surgical tool to the surgical tool-fixing device for a surgical robot according to the first embodiment of the disclosure, in which FIG. 6A shows the cutter having a head diameter of 3.2 mm, FIG. 6B shows the cutter having a head diameter of 5.0 mm, and FIG. 6C shows the cutter having a head diameter of 6.2 mm. FIGS. 7A and 7B are perspective views showing states that a cutter applied as a surgical tool to the surgical tool-fixing device for a surgical robot according to the first embodiment of the disclosure, in which FIG. 7A shows an installation state of the cutter having a head diameter of 5.0 mm, and FIG. 7B shows an installation state of the cutter having a head diameter of 6.2 mm.

Referring to FIGS. 6A to 7B, a cutter 7a may be selected and used according to the surgical methods as shown in FIGS. 6A to 6c among a cutter 7a having a head diameter of 3.2 mm, a cutter 7a' having a head diameter of 5.0 mm, and a cutter 7a" having a head diameter of 6.2 mm.

In more detail, among artificial knee joint surgeries, total knee arthroplasty (TKA) that cuts both sides of femur and tibia usually employs the cutter 7a" with a relatively long shaft having a head diameter of 6.2 mm, and Uni-knee Arthroplasty(UKA) that cuts only one side of the femur and tibia employs a cutter 7a' with a relatively short shaft having a head diameter of 5.0 mm, and guide hole resection (GHR) that performs cutting (sawing) during surgery by fixing a cutting block to the bone with a fixing pin for which an insertion hole is formed using a cutter 7a having a head diameter of 3.2 mm. The sleeve 1 according to the disclosure, allows various types of cutters mentioned above to be interchangeably installed thereto, thereby having the advantages of performing surgical preparation, surgery, etc., conveniently and quickly.

Although the cutters 7a, 7a' and 7a" described above are different in the outer diameter and shape of a cutter head 714 and have the same or different overall length of the shaft 71, the shafts of the cutters 7a, 7a' and 7a" include a small diameter portion 711 shaped like a round bar and having a relatively small diameter so as to be inserted in the chuck device part 2 in common.

The cutter 7a having a head diameter of 3.2 mm, the cutter 7a' having a head diameter of 5.0 mm, and the cutter 7a" having a head diameter of 6.2 mm are formed with a mounting large diameter portion 712 being in contact with the small diameter portion to be inserted in the sleeve 1 and having a relatively large outer diameter compared to the small diameter portion, and a medium diameter portion 713 extending being in contact with the mounting large diameter portion 712, formed with a cutter head 714 at an end portion thereof, and having an outer diameter larger than that of the small diameter portion and smaller than that of the mounting large diameter portion.

In addition, the cutter heads 714 of the cutters 7a, 7a' and 7a" have two, three, and four blades, respectively, having helix angles of 20 to 40 degrees, and the foregoing cutters may be used appropriately for each surgical technique in such a manner that the cutter having a head diameter of 3.2 mm and the cutter having a head diameter of 5.0 mm are formed to have a protrusion length of 40 mm to 70 mm from a free end, i.e., the other end of the sleeve 1 in the mounted state, and the cutter having a head diameter of 6.2 mm is formed to have a protrusion length of 70 mm to 85 mm.

Further, the foregoing cutters are marked with two mounting checking bands 715 on the mounting large diameter portion 712 so as to check whether they are mounted or not, without using a separate cutter gauge. In this case, the outer one of the two mounting checking bands 715 is marked to be exposed at a point when the cutter 7a, 7a' or 7a" is normally mounted. Therefore, when the outer one of the two mounting checking bands 715 is exposed, it is confirmed that the cutter 7a, 7a' or 7a" is normally mounted. On the other hand, when both the two mounting checking bands 715 are not visible or both are visible, it is confirmed that the cutter 7a, 7a' or 7a" is abnormally mounted, and thus the mounting state of the cutter should be adjusted to expose the one mounting checking band before use.

As described above, the foregoing cutters are provided with the mounting large diameter portion 712 so as to be installed in the shaft insertion hole 12 of the sleeve 1, thereby being mounted conveniently to a single surgical device and used interchangeably as shown in FIGS. 7A and 7B.

The operations of the surgical tool-fixing device for a surgical robot according to the first embodiment of the disclosure will be briefly described below.

In the foregoing surgical tool-fixing device, the motor 9 is connected to the motor connecting member 37 of the holder head 32, the arm connecting member 34 is coupled to the clamping member (not shown) provided in the robot arm (not shown) of the orthopedic surgical robot, and then the robot arm of the surgical robot operating based on information input to a computer positions the surgical tool-fixing device to a surgical site.

In this state, when the motor operates based on the information input to the computer, the cutter 7a clamped as the surgical tool 7 to the chuck device part 3 rotates to cut a set surgical area of the patella. With this cutting process, a procedure is performed by mounting an artificial knee joint (implant) in a given order.

Further, the shaft 71 is supported by the sleeve 1 and rotates stably while the cutter 7a rotates according to the rotation of the motor. In this case, the sleeve 1 is not conventionally screw-coupled to the chuck body, but the first sleeve fastening portion 18 formed in the sleeve 1 is screw-coupled to the second sleeve fastening portion 38 formed on the holder part 3, thereby not only maintaining a firmly holding state but also fundamentally preventing an external force from being applied to the chuck device part 2.

In addition, as shown in FIG. 5, the end surface of the sleeve is in surface contact with the sleeve mounting portion 352 and thus has a non-swaying close-contact structure. Therefore, the conventional separation distance d (see FIG. 1A) is not formed between the sleeve and the holder head, and thus slight left-right deformation is not induced even though the surgical tool is moved to the left or right with force in the cutting process.

Accordingly, the shaft 71 of the cutter 7a, 7a', 7a", the built-in bearings of the sleeve 1, etc. are prevented from deformation and improved in durability, and the chuck device part 2 to which the shaft 71 is clamped is also prevented from deformation and improved in durability, thereby reducing maintenance costs, performing surgery stably, lowering a risk of negligent accidents, and shortening downtime of surgery.

Meanwhile, when the surgical tool-fixing device for a surgical robot according to the disclosure is to be disassembled and assembled for cleaning or replacing the cutter, i.e., the surgical tool, a user can easily perform the clamping operation and the unclamping operation of the chuck device part 2 for the cutter shaft 71 by holding the first and second handles 3612 and 3622 protruding from the operating handle 36 and rotating them 180° in the forward direction and 180° in the reverse direction, and even a female nurse who has a weak grip can conveniently perform the operations without difficulty.

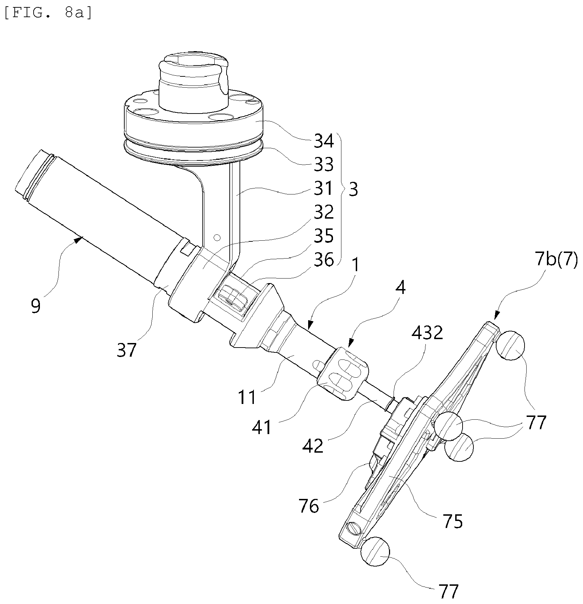

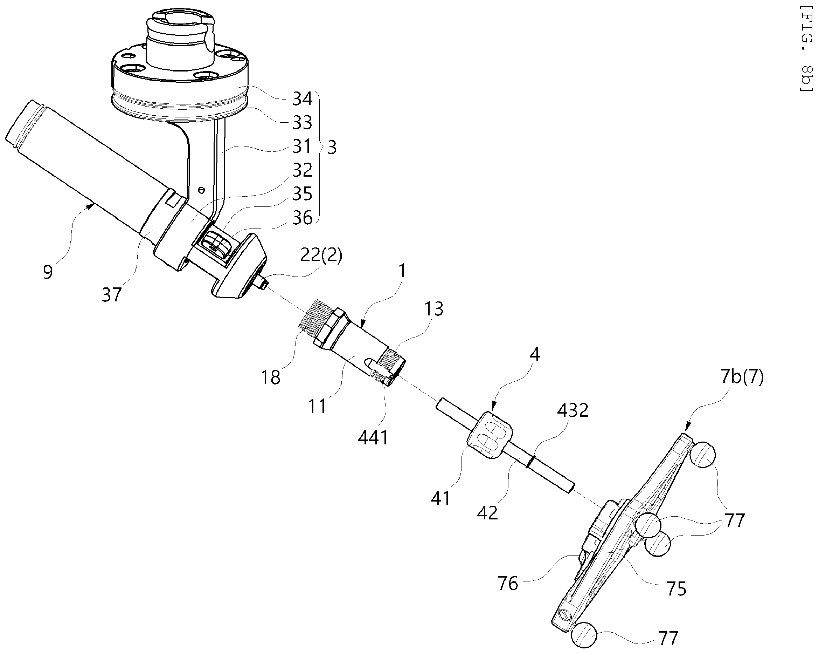

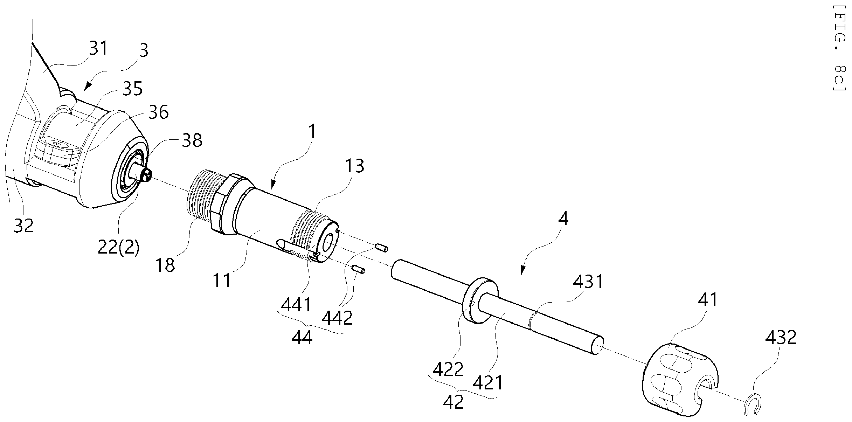

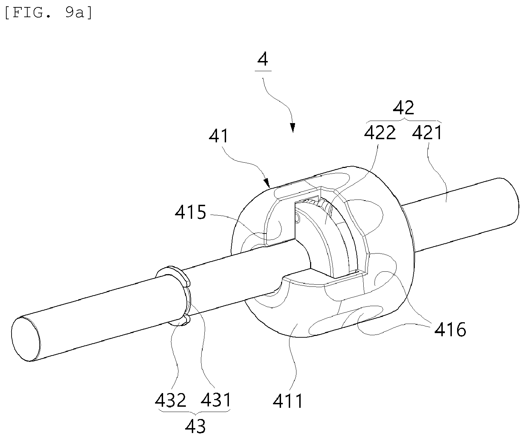

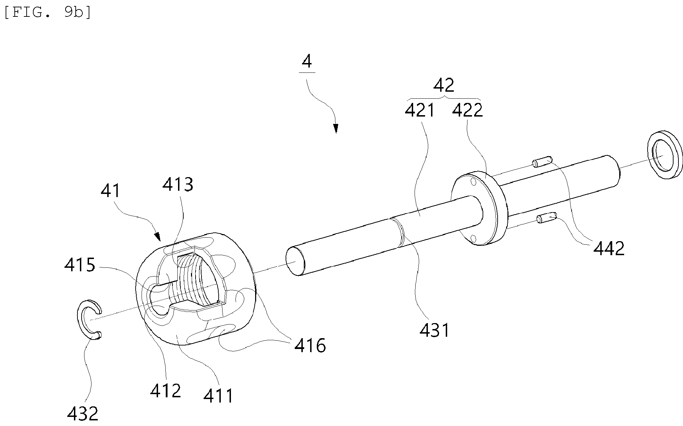

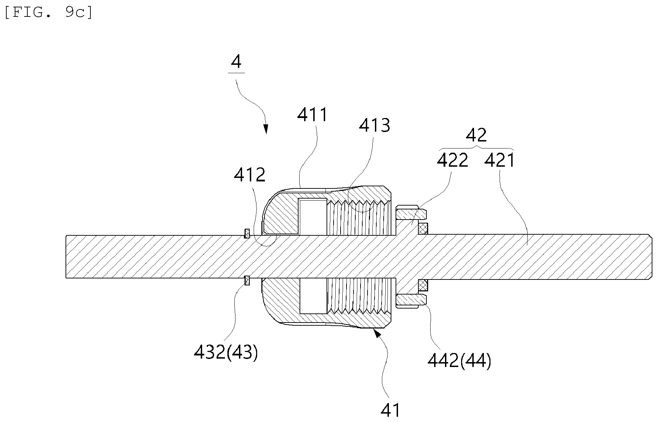

FIGS. 8A to 8C are views showing states that a calibration tool is installed as a surgical tool in the surgical tool-fixing device for a surgical robot according to the first embodiment of the disclosure, in which FIG. 8A is a perspective view, FIG. 8B is a schematic exploded perspective view, and FIG. 8C is an exploded perspective view of a main component. FIGS. 9A to 9C are views to illustrate a calibration mounting part installed in the surgical tool-fixing device for a surgical robot according to the first embodiment of the disclosure, in which FIG. 9A is a partial perspective view, FIG. 9B is a partially exploded perspective view, and FIG. 9C is a partial cross-sectional view.

Referring to FIGS. 9A to 9C, the calibration mounting part 4 is detachably provided in the sleeve 1 so that the calibration tool 7b such as the marker for calibrating the tip of the cutter (or drill) can be installed in artificial joint surgery using a robot.

In more detail, the calibration mounting part 4 includes a holding cap member 41 fastened to the sleeve 1, and a calibration mounting member 42 mounted to the sleeve 1 by the holding cap member 41.

The calibration mounting member 42 includes a calibration shaft 421 in which the marker is installed, and a position setting member 422 formed in the calibration shaft 421 and seated on the holding cap member 41.

The calibration shaft 421 is formed in a bar-shaped structure, such as a round bar, having a predetermined length. According to this embodiment, the calibration may be easily performed using one calibration shaft 421 because a cutter having a head diameter of 5.0 mm for Uni-knee Arthroplasty (UKA) and a cutter having a head diameter of 3.2 mm for Guide Hole Resection (GHR) have the same overall length of 115.85 mm. However, a cutter having a head diameter of 6.2 mm for Total Knee Arthroplasty (TKA) has a longer length of 135.65 mm, and therefore the calibration may be performed using the same calibration shaft by inputting a value compensated for length difference to a robot system in advance to correct calibration data of the cutter of 6.2 mm.

The position setting member 422 is formed on the outer circumferential surface of the calibration shaft 421, and shaped like a disc to be inserted and seated inside the holding cap member 41.

As shown in FIG. 9B, the holding cap member 41 includes a cap body 411 shaped like a cap with an anti-slip groove 416 recessed thereon and connected to a hollow sleeve body 11, a rod insertion hole 412 formed penetrating the cap body to insert the calibration shaft 421 therein, and a cap female thread 413 formed on the inner circumferential surface of the cap body to be fastened to the coupling male-thread portion 13 of the sleeve 1.

In addition, the holding cap member 41 may be formed with an inspection opening 415 to inspect the introduction of foreign substances by checking the alignment state between the sleeve 1 and the cap body 411.

For example, the inspection opening 415 is formed by cutting about 1/4 out of the cap body 411, and shows the interior to check the alignment state of the calibration mounting member 42. In other words, a gap formed when foreign substances are introduced between the inner surface of the position setting member 422 and the end of the sleeve 1 is checkable with the naked eye through the inspection opening 415. When the gap is confirmed with the naked eye, the holding cap member 41 is separated from the sleeve, cleaned, and then reassembled, thereby performing the calibration accurately.

Further, the calibration mounting member 42 includes a cap separation preventing means 43 to prevent the holding cap member 41 from being separated from the calibration shaft 421.

The cap separation preventing means 43 may be variously configured without any particular limitations as long as it can prevent the separation of the holding cap member 41. According to this embodiment, the cap separation preventing means 43 includes a separation-preventing groove 431 recessed on the calibration shaft 421, and a separation-preventing piece 432 such as a C-ring to be press-fitted into the separation-preventing groove.

Meanwhile, the calibration mounting part 4 includes a mounting member rotation blocking portion 44 configured to block the movement of the calibration mounting member 42 for accurate and stable calibration.

The mounting member rotation blocking portion 44 includes a pin insertion groove 441 formed in the sleeve 1, and a rotation blocking pin 442 formed in the position setting member and inserted in the pin insertion groove 441.

Here, the rotation blocking pin 442 includes two bar-shaped pins installed protruding from the position setting member 422 at an angle of 180°.

In addition, the pin insertion groove 441 is formed in the sleeve 1 at a position corresponding to the rotation blocking pin 442, and structured to have a foreign substance discharging portion on one side to facilitate the discharge of the foreign substances. Here, the foreign substance discharging portion refers to a portion exposed to the outside by cutting an outer portion out of the pin insertion groove 441.

Meanwhile, the calibration tool 7b refers to an optical marker detachably provided in the sleeve 1 and applied to an Optical Tracking System (OTS), and includes a marker body 75 formed with a shaft holding hole (not shown) in which the calibration shaft 421 is inserted, and a tightening means 76 such as a fastening bolt to secure the calibration shaft 421 inserted in the shaft holding hole. In addition, the marker body 75 includes a plurality of position transmitting units 77 roughly shaped like balls to reflect or transmit a position signal to the OTS. For reference, the OTS refers to a system that tracks the marker with a plurality of infrared cameras, and converts the distance through triangulation, thereby tracking the position and posture in a three-dimensional space in real-time. The tracking principle of the OTS has been widespread, and thus detailed descriptions thereof will be omitted for simplicity of description.

Below, a process of installing and using the calibration tool as the surgical tool in the surgical tool-fixing device for a surgical robot according to the disclosure will be briefly described with reference to FIGS. 8A and 8B.

As described above, the cutter having a head diameter of 3.2 mm, the cutter having a head diameter of 5.0 mm, or the cutter having a head diameter of 6.2 mm may be selectively installed according to the surgical method, and then the calibration mounting part 4 may be used to easily install the calibration tool 7b such as the marker for calibrating the tip of the cutter during the artificial joint surgery using the robot.

In more detail, the calibration mounting part 4 is assembled in such a manner that the cutter 7a is first separated from the chuck device part 2, the calibration mounting member 42 is inserted in the holding cap member 41, and the holding cap member 41 is fastened to the coupling male-thread portion 13 of the sleeve 1. In this assembling process, when the rotation blocking pin 442 formed in the position setting member 422 is inserted in the pin insertion groove 441 formed in the sleeve 1, the movement such as the rotation of the calibration mounting member 42 in which the calibration tool 7b is installed is fundamentally blocked, thereby having an advantage of enabling accurate calibration.

In addition, the foreign substance discharging portion, i.e., the exposed portion is formed by cutting an outer portion out of the pin insertion groove 441 formed in the sleeve 1, thereby having an advantage of easily discharging foreign substances to the outside even though the foreign substances are introduced due to the operation of the cutter during the surgery.

Further, the inspection opening 415 is formed in the holding cap member 41, and thus, when a gap formed by foreign substances introduced between the inner surface of the position setting member 422 and the end of the sleeve 1 is confirmed with the naked eye, the holding cap member 41 is separated from the sleeve, cleaned, and then reassembled, thereby performing the calibration accurately.

### [MODE FOR INVENTION]

Below, other embodiments of the disclosure will be described, in which detailed descriptions about components similar to those shown in the first embodiment will be omitted and descriptions will be made focusing on components having differences. Further, the embodiments set forth herein may selectively employ the components of the first embodiment or the components of other embodiments if their structures are applicable thereto, and detailed descriptions or drawings thereof will be omitted.

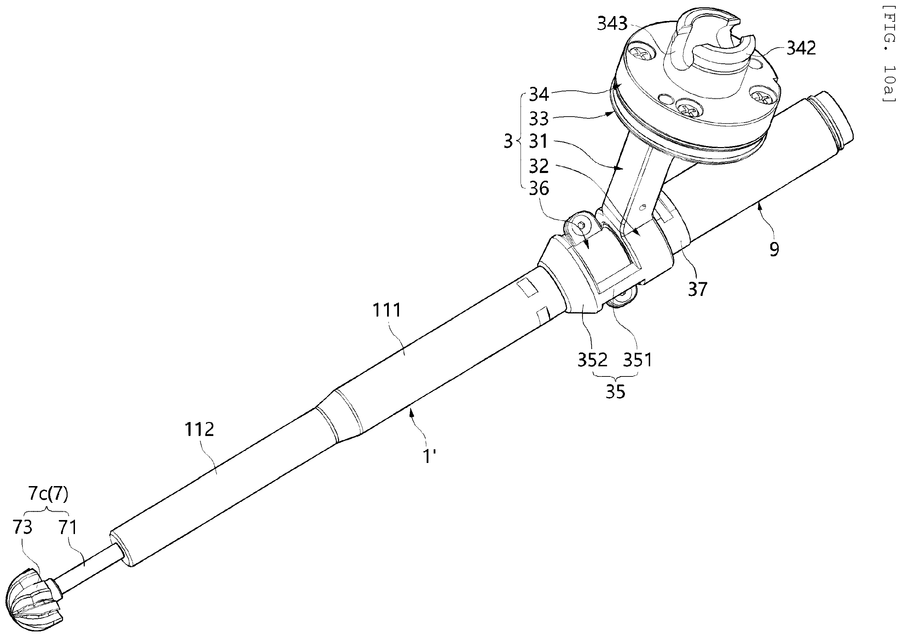

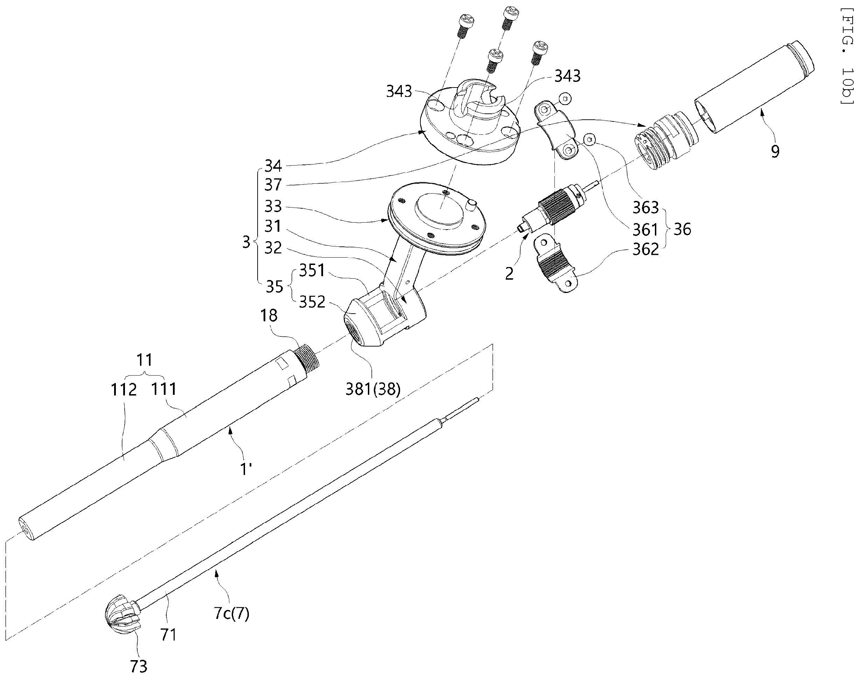

FIGS. 10A and 10B are views to illustrate a surgical tool-fixing device for a surgical robot according to a second embodiment of the disclosure, in which FIG. 10A is a perspective view, and FIG. 10B is an exploded perspective view.

Referring to FIGS. 10A and 10B, the surgical tool-(fixing) device for a surgical robot according to the second embodiment of the disclosure includes a sleeve 1' having the first sleeve fastening portion 18, and the holder part 3 having the second sleeve fastening portion 38, so that the sleeve 1' can be directly coupled to the holder part 3, in which the sleeve 1' is configured to be mounted with a reamer used for cutting the acetabulum of a hip joint during artificial hip joint surgery.

To this end, the sleeve 1' includes a hollow sleeve body 11' having a shaft insertion hole formed penetratingly along the longitudinal direction, and the hollow sleeve body 11' includes a first hollow sleeve body 111 having the first sleeve fastening portion 18 formed at the end thereof, and a second hollow sleeve body 112 extending in a rod-shaped structure with a smaller outer diameter than the first hollow sleeve body 111, in which the first hollow sleeve body 111 and the second hollow sleeve body 112 are formed as a single body.

The foregoing surgical tool 7 is provided as a reamer 7c including a reamer basket-cup 73 that is formed at the end of the shaft 71 to be inserted into the shaft insertion hole and performs a cutting operation.

The reamer basket-cup 73 has two or more blades, is approximately shaped like a semi-sphere shape suitable for cutting the acetabulum, and is formed having a diameter ranging from 10 to 40 mm.

In addition, the reamer 7c is configured to have a protrusion length of 2 to 10 cm from the free end of the sleeve 1 in the mounted state.

Meanwhile, the surgical tool-fixing device for a surgical robot according to the second embodiment of the disclosure is mounted with the reamer 7c as the surgical tool, positions the reamer 7c to a surgical site, and then drives the motor 9, thereby causing the reamer basket-cup 73 to rotate and cut the acetabulum of the hip joint.

When pretreatment procedures such as cutting are completed, the reamer 7c is separated, and an impactor (not shown) is used to press-fit and fix an artificial acetabulum cup into the cut acetabulum and connect an artificial femoral head according to a typical artificial hip joint surgery method.

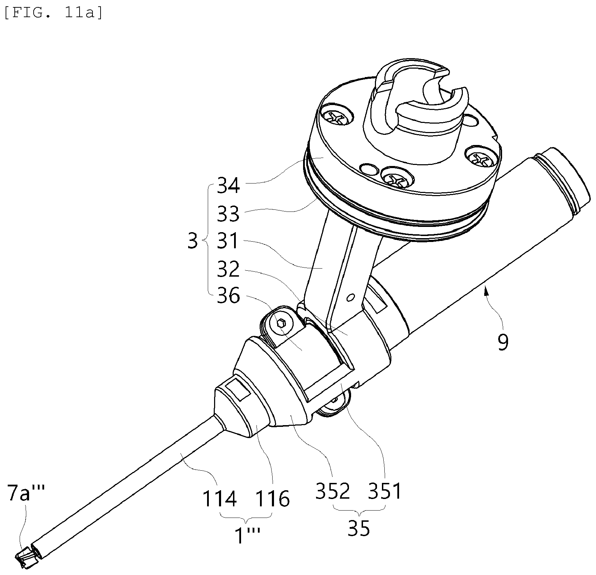

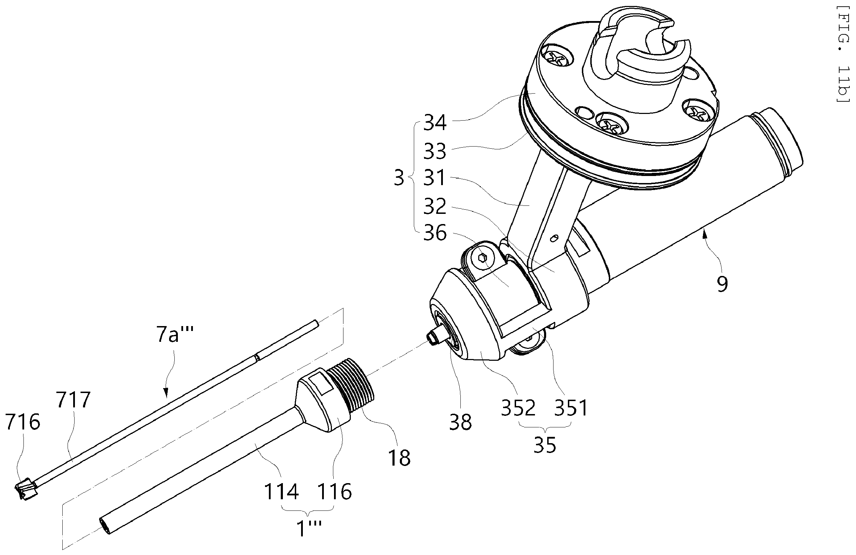

FIGS. 11A and 11B are views to illustrate a surgical tool-fixing device for a surgical robot according to a third embodiment of the disclosure, in which FIG. 11A is a perspective view, and FIG. 11B is an exploded perspective view of a sleeve portion having distinctive features.

Referring to FIGS. 11A and 11B, the surgical tool-fixing device for a surgical robot according to the third embodiment of the disclosure includes a sleeve 1" having the first sleeve fastening portion 18, and the holder part 3 having the second sleeve fastening portion 38, so that the sleeve 1" can be directly coupled to the holder part 3, in which the sleeve 1" includes a pipe portion 114 shaped like a pipe and having a small diameter, and a fastening cap portion 116 formed at one end of the pipe portion to be fastened to the chuck device part 2.

The pipe portion 114 includes bearings (not shown), a spacer (not shown), etc., inserted therein to rotatably support the shaft of the drill.

The fastening cap portion 116 is shaped like a cap formed with a shaft insertion hole along the longitudinal direction, and includes the first sleeve fastening portion 18 formed at an end portion thereof facing the second sleeve fastening portion.

A cutter 7a‴ includes a cutter head 716 formed with a cutting blade, and a shaft 717 shaped like a round bar and formed extending from the cutter head 716.

Meanwhile, in the surgical tool-fixing device for a surgical robot according to the third embodiment of the disclosure, as shown in FIG. 11A, when the motor operates based on the information input to the computer, like that of the first embodiment, the cutter 7a‴ clamped as the surgical tool 7 to the chuck device part 3 rotates to cut a set surgical area of the patella. With this cutting process, a procedure is performed by mounting an artificial knee joint (implant) in a given order.

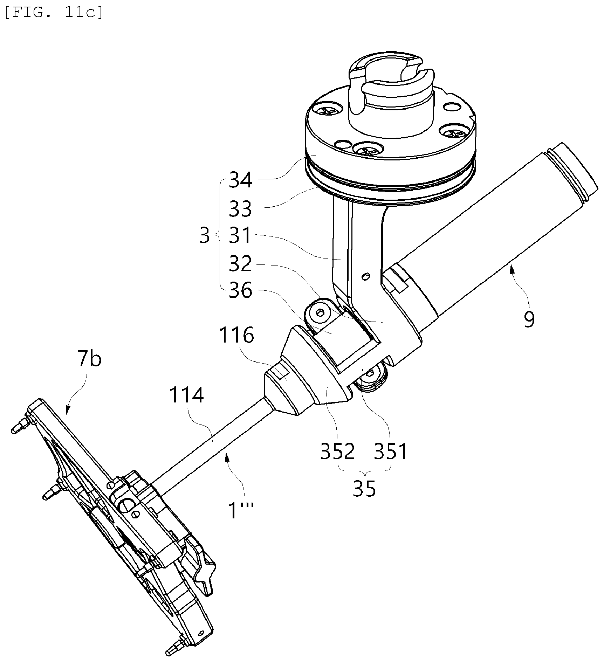

FIG. 11C is a view showing a state in which a calibration tool is mounted to the sleeve of the surgical tool-fixing device for a surgical robot according to the second embodiment of the disclosure. As shown therein, when the tip of the cutter 7a‴ is to be calibrated in the artificial joint surgery using a robot, the calibration may be easily performed by installing the calibration tool 7b to the end portion of the pipe portion 114 of the sleeve 1".

The terms "comprise," "configure," and/or "have" specify the presence of stated components, unless there is a clearly different meaning in the disclosure, but do not preclude the presence thereof and should be construed to further include other components. Unless otherwise defined, all terms, including technical or scientific terms used herein, have the same meaning as commonly understood by those skilled in the art to which the disclosure pertains. General terms that are defined in a dictionary shall be construed to have meanings that are consistent in the context of the relevant art, and will not be interpreted as having an idealistic or excessively formalistic meaning unless clearly defined in the disclosure.

Although the configurations and operations of a surgical tool-fixing device for a surgical robot according to the first embodiment of the disclosure have been described above, it will be understood by a person having ordinary knowledge in the art that the embodiments are merely illustrative and may be partially substituted and modified without departing from the scope of the disclosure

Accordingly, the scope of the disclosure should be understood to extend to the appended claims and their equivalents.

### [INDUSTRIAL APPLICABILITY]

The disclosure relates to a surgical tool-fixing device for a surgical robot to stably hold and support a sleeve for supporting a shaft of a surgical tool during an artificial joint surgery using a robot, which may be applied even to surgeries using no robot, and may be used to hold and support the surgical tool various surgeries in addition to the artificial joint surgery.

## Claims

1. A surgical tool-fixing device for a surgical robot, comprising:
a sleeve supporting a surgical tool comprising a shaft;
a chuck device part comprising a chuck body to which the sleeve is coupled, chucks selectively provided inside the chuck body and selectively clamping the shaft to hold the surgical tool, and an operation force applying portion applying operation forces for clamping and unclamping operations of the chucks; and
a holder part installing the chuck device part thereon, mounted to a distal end of a robot arm, and comprising a sleeve supporting portion holding and supporting the sleeve not to shake, wherein
the sleeve comprises a first sleeve fastening portion formed in a direction facing the sleeve supporting portion of a hollow sleeve body formed with a shaft insertion hole, and
the sleeve supporting portion is formed with a second sleeve fastening portion to couple with the first sleeve fastening portion.

2. The surgical tool-fixing device of claim **1,** wherein
the first sleeve fastening portion comprises a sleeve male thread portion comprising a plurality of male threads formed on an outer circumferential surface of a thread-formed hollow body integrally formed on a first side of the hollow sleeve body,
the sleeve supporting portion comprises a supporting protrusion extending from the holder part, and a sleeve mounting portion formed at an end portion of the supporting protrusion, and
the second sleeve fastening portion comprises a holder female thread portion formed with a plurality of female threads on the sleeve mounting portion to couple with the sleeve male thread portion.

3. The surgical tool-fixing device of claim **2,** wherein
the supporting protrusion protrudes forward to provide an operational space inside which the operation force applying portion is positioned, and
the sleeve mounting portion is formed with the holder female thread portion on an inner circumferential surface of a hollow hole of a conical body with a small front outer diameter and a large rear outer diameter.

4. The surgical tool-fixing device of claim **2,** wherein
the hollow sleeve body is structured to comprise a first hollow sleeve body formed with the first sleeve fastening portion at an end portion thereof, and a second hollow sleeve body extending in a rod-shaped structure with a smaller outer diameter than the first hollow sleeve body, and
the surgical tool comprises a reamer with a reamer basket-cup formed at an end portion of the shaft to be inserted in the shaft insertion hole and performing a cutting operation.

5. The surgical tool-fixing device of claim **2,** further comprising a calibration mounting part that comprises a holding cap member fastened to the hollow sleeve body to install a calibration tool, and a calibration mounting member mounted to the sleeve by the holding cap member.

6. The surgical tool-fixing device of claim **5,** wherein
the calibration mounting member comprises a bar-shaped calibration shaft in which a marker is installed, and a position setting member formed in the calibration shaft and seated on the holding cap member,
the holding cap member comprises a cap body shaped like a cap, a rod insertion hole formed in the cap body to insert the calibration shaft therein, and a cap female thread formed on an inner circumferential surface of the cap body to be fastened to a cap-coupling male thread formed on a second side of the hollow sleeve body, and
the surgical tool-fixing device further comprises a mounting member rotation blocking portion configured to block movement of the calibration mounting member.

7. The surgical tool-fixing device of claim 2, wherein the surgical tool comprises a plurality of cutters comprising a cutter head with a plurality of blades on an outer end portion of the shaft, in which the cutter having a head diameter of 3.2 mm and the cutter having a head diameter of 5.0 mm are formed to have a protrusion length of 40 mm to 70 mm from a free end of the sleeve in a mounted state, and the cutter having a head diameter of 6.2 mm is formed to have a protrusion length of 70 mm to 85 mm.

8. The surgical tool-fixing device of any one of claims 1 to 7, further comprising an operating handle connecting with the operation force applying portion and applying an operating force,
wherein the operating handle comprises an engagement portion placed in the operational space to engage with the operation force applying portion, and a handle unit extending from the engagement portion.

9. The surgical tool-fixing device of claim **8,** wherein the handle unit comprises a first handle unit comprising a first engagement portion to engage with an outer surface of the operation force applying portion on a first side, and first handles extending from both ends of the first engagement portion and formed with fastening holes; a second handle unit comprising a second engagement portion to engage with an outer surface of the operation force applying portion on a second side, and second handles extending from both ends of the second engagement portion and formed with fastening holes; and fastening members to be fastened to the fastening holes of the first and second handle units, so as to be placed and assembled inside the operational space.
